# EUROPÄISCHE PATENTSCHRIFT

(11) **EP 4 011 511 B1**
(45) Veröffentlichungstag und Bekanntmachung des Hinweises auf die Patenterteilung: **28.06.2023**
(21) Anmeldenummer: 20213352.6
(22) Anmeldetag: 11.12.2020
(51) Int. Cl.: B08B 3/02

(54) **REINIGUNGSANLAGE, FÖRDEREINRICHTUNG UND SEPARIERUNGSEINRICHTUNG FÜR INSEKTEN ODER WÜRMER UND ENTSPRECHENDE VERFAHREN**
CLEANING SYSTEM, CONVEYING DEVICE AND SEPARATING DEVICE FOR INSECTS OR WORMS AND CORRESPONDING METHOD
INSTALLATION DE NETTOYAGE, DISPOSITIF DE TRANSPORT ET DISPOSITIF DE SÉPARATION POUR INSECTES OU VERS ET PROCÉDÉ CORRESPONDANT

(43) Veröffentlichungstag der Anmeldung: 15.06.2022
(73) Patentinhaber: Bühler AG, 9240 Uzwil (CH)
(72) Erfinder: DE WOLF, Luc, 5701 PT Helmond (NL); AARTS, Kees Wilhelmus Petrus, 5262 Vught (NL); HARMS, Stijn, 5212 AM Hertogenbosch (NL); JANSEN, Maurits Petrus Maria, 4854 CA Bavel (NL)
(74) Vertreter: Vossius & Partner Patentanwälte Rechtsanwälte mbB

(56) Entgegenhaltungen:
- WO-A1-2020/011903
- DE-B3-102016 115 189
- DE-U1-202016 104 502

## Beschreibung

Die vorliegende Offenbarung befasst sich mit der Verarbeitung bzw. Behandlung von Insektenlarven. Insbesondere umfasst die vorliegende Offenbarung eine Reinigungsanlage zur Reinigung von lebenden Insekten, insbesondere von lebenden Insektenlarven, oder von lebenden Würmern, ein Verfahren unter Verwendung einer derartigen Reinigungsanlage, eine Fördereinrichtung sowie eine Reinigungseinrichtung.

Die Erfindung betrifft das Gebiet der Gewinnung von Nährstoffen, Futtermitteln und Nahrungsmitteln aus Insekten oder Würmern.

In den letzten Jahrzehnten hat das Interesse an der Verwendung von Insekten und Würmern als Lebensmittel- und Futtermittelquelle zugenommen, insbesondere angesichts des Wachstums der Weltbevölkerung und des steigenden Bedarfs an alternativen und nachhaltigen Proteinquellen für die Nutztierindustrie. Da Insekten und Würmer üblicherweise reich an Proteinen und Fetten sind, weisen sie einen relativ hohen Nähr- bzw. Kalorienwert auf und sind dementsprechend besonders gut geeignet für die menschliche Ernährung sowie die Nutztierzucht.

Es ist im ökonomischen und ökologischen Sinne wünschenswert, Insekten und Würmer im industriellen Maßstab züchten und verarbeiten zu können, um standardisiert Nährstoffe zu produzieren, die anschliessend zur Herstellung von Lebens- oder Futtermitteln verwendet werden können. Wichtig bei der Verarbeitung ist eine schonende Handhabung der noch lebenden Insekten und die Herstellung von sicheren Endprodukten.

Um möglichst saubere Larven ohne anhaftende Resten aus dem Züchtungsprozess zu erhalten, erfolgt in der Verarbeitungskette zum Teil ein Waschschritt mit Wasser. Nachteilig an bekannten Verfahren und Anlagen ist, dass bei der Separierung von Insekten und Wasser die Siebeffizienz oftmals nicht ausreichend ist. Des Weiteren ist der Wasserverbrauch oftmals hoch und ein schonender Transport der Insekten sowie ein effizientes Waschen, d.h. eine ausreichende Entfernung von Resten aus dem Züchtungsprozess, nicht immer gegeben. In herkömmlichen Anlagen kann es ferner dazu kommen, dass lebende Larven oder Würmer durch die Sieböffnungen kriechen. DE 20 2016 104502 U1 betrifft eine Reinigungsvorrichtung für Insektenschutzgitter. Diese weist eine erste Fördereinrichtung auf, welche die zu reinigenden Insektenschutzgitter einer Reinigungseinheit zuführt, wobei die Reinigungseinheit mit Düsen zur Zuführung und Verteilung von Reinigungsflüssigkeit auf die zu reinigenden Insektenschutzgitter ausgestattet ist, und die Reinigungseinheit mit einer Rollen-/Walzenanordnung ausgestattet ist, bei der wenigstens ein, aus an beiden Seiten des Insektenschutzgitters angeordneten, gegenläufig drehenden Reinigungsrollen gebildetes, Rollenpaar am Gewebe des Insektenschutzgitters zum Anliegen kommt.

DE 10 2016 115189 B3 bezieht sich auf eine der DE 20 2016 104502 U1 ähnliche Reinigungsvorrichtung für Insektenschutzgitter.

Es ist eine Aufgabe der vorliegenden Erfindung, die aus dem Stand der Technik bekannten Nachteile zu überwinden. Ferner ist eine Aufgabe der vorliegenden Erfindung, eine Reinigungsanlage zur Reinigung von lebenden Insekten oder Würmern sowie ein entsprechendes Verfahren zur Reinigung von lebenden Insekten oder Würmern bereitzustellen, die eine schonende und stressfreie Behandlung bzw. Verarbeitung der lebenden Insekten oder Würmer gewährleisten sowie eine Wasserwiederverwendung oder - aufbereitung beinhalten. Weiterhin soll durch die Möglichkeit zur Lagerung von lebenden Insekten oder Würmern in Wasser die Züchtung der Insekten von der weiteren Verarbeitung entkoppelt werden, wodurch beide Bereiche unabhängig voneinander betrieben werden können und die Flexibilität in der Anlage erhöht wird. Wenn im Folgenden von "Insekten" oder "Larven" die Rede ist, sollen auch ohne ausdrückliche Erwähnung Insekten, Larven oder Würmer als mit eingeschlossen verstanden werden.

Diese Aufgaben werden durch die Merkmale der unabhängigen Ansprüche gelöst. Weitere vorteilhafte Ausführungen ergeben sich aus Kombinationen mit den Merkmalen der entsprechenden Unteransprüche sowie aus den Ausführungen in der Beschreibung und den Figuren.

Die vorliegende Erfindung betrifft eine Reinigungsanlage zur Reinigung von lebenden Insekten, insbesondere lebenden Insektenlarven, oder Würmern gemäß Anspruch 1 sowie deren Verwendung gemäß Anspruch 7. Weiter betrifft die vorliegende Erfindung eine Fördereinrichtung gemäß Anspruch 9 sowie eine Larvenseparierungseinrichtung gemäß Anspruch 10. Die Erfindung ist in den unabhängigen Ansprüchen definiert. Abhängige Ansprüche beschreiben bevorzugte Ausführungsformen.

Insbesondere betrifft die vorliegende Offenbarung eine Reinigungsanlage zur Reinigung von lebenden Insekten, insbesondere von lebenden Insektenlarven, oder von lebenden Würmern umfassend eine Sammeleinrichtung zur Aufnahme und zur Vermischung der Insekten oder der Würmer mit Wasser zu einer Mischung, eine erste Fördereinrichtung zum Fördern der Mischung aus der Sammeleinrichtung und eine erste Larvenseparierungseinrichtung zur Separierung der Insekten oder der Würmer vom Wasser aus der von der ersten Fördereinrichtung geförderten Mischung. Das separierte Wasser steht zur weiteren Verwendung in einer Insektenanlage, insbesondere der Reinigungsanlage zur Verfügung, insbesondere wird es zumindest teilweise in der Reinigungsanlage rezirkuliert.

Verschiedene Ausführungsformen können ferner folgende Merkmale umfassen.

Die Reinigungsanlage weist ferner bevorzugt zumindest einen Speichertank zur Massenspeicherung der Mischung auf, wobei der zumindest eine Speichertank bevorzugt nach der ersten Fördereinrichtung angeordnet ist, wobei die Reinigungsanlage vorzugsweise ferner zumindest eine weitere Fördereinrichtung zum Fördern der Mischung aufweist. Die zumindest eine weitere Fördereinrichtung ist vorzugsweise zwischen der ersten Fördereinrichtung und dem Speichertank oder nach dem Speichertank angeordnet.

Ferner kann eine weitere Sammeleinrichtung zur Aufnahme und zur Vermischung der Insekten oder der Würmer mit Wasser zu einer Mischung vorgesehen sein, wobei die zumindest eine weitere Sammeleinrichtung vorzugsweise nach der ersten Larvenseparierungseinrichtung angeordnet ist.

Die Reinigungsanlage weist ferner bevorzugt zumindest eine weitere Larvenseparierungseinrichtung zur weiteren Separierung der Insekten oder der Würmer vom Wasser aus der geförderten Mischung auf. Vorzugsweise steht das separierte Wasser zur weiteren Verwendung in der Insektenanlage, insbesondere in der Reinigungsanlage zur Verfügung, insbesondere wird es zumindest teilweise in der Reinigungsanlage rezirkuliert. Die zumindest eine weitere Larvenseparierungseinrichtung ist vorzugsweise nach der ersten Larvenseparierungseinrichtung angeordnet.

Zumindest eine weitere Wasserzuführung zur Nachwässerung der Mischung kann vorgesehen sein, die vorzugsweise nach der ersten Larvenseparierungseinrichtung in den Kreislauf und/oder in die weitere Sammeleinrichtung und/oder in den zumindest einen Speichertank mündet.

Vorzugsweise ist zumindest ein Wassertank zur Speicherung von Wasser aus und zur Abgabe von Wasser in den Kreislauf vorgesehen.

Vorzugsweise ist zumindest eine Filtereinrichtung zur Filterung des separierten Wassers aus der ersten Larvenseparierungseinrichtung und/oder der zumindest einen weiteren Larvenseparierungseinrichtung vorgesehen.

Vorzugsweise ist zumindest eine Temperiereinrichtung zur Temperierung des Wassers vorgesehen, wobei die zumindest eine Temperiereinrichtung vorzugsweise zwischen dem zumindest einen Wassertank und der Sammeleinrichtung oder an dem Wassertank angeordnet ist.

Vorzugsweise ist zumindest eine Wiegeeinrichtung zur Bestimmung des Gewichts der Insekten oder Würmer vorgesehen, wobei die Wiegeeinrichtung vorzugsweise vor der Sammeleinrichtung oder in der Sammeleinrichtung integriert angeordnet ist.

Ferner umfasst die Offenbarung ein Verfahren zur Reinigung von lebenden Insekten, insbesondere von lebenden Insektenlarven, oder von lebenden Würmern mit einer wie oben beschriebenen Reinigungsanlage. Das Verfahren umfasst die Schritte:
- Zuführen der lebenden Insekten oder lebenden Würmer in die Sammeleinrichtung;
- erstes Vermischen der lebenden Insekten oder lebenden Würmer mit Wasser, bis eine bestimmte Mischung erreicht ist;
- Fördern der Mischung zu der ersten Larvenseparierungseinrichtung mittels der ersten Fördereinrichtung;
- erstes Separieren der Mischung in der ersten Larvenseparierungseinrichtung;
- Zumindest partielles Bereitstellen bzw. Rezirkulieren des separierten Wassers für die weitere Verwendung in der Insektenanlage, insbesondere der Reinigungsanlage.

Verschiedene Ausführungsformen können ferner folgende Merkmale umfassen.

Das Verfahren weist ferner bevorzugt das Fördern der in der ersten Larvenseparierungseinrichtung separierten Mischung zu dem zumindest einen Speichertank mittels der zweiten Fördereinrichtung und das Speichern der Mischung in dem zumindest einen Speichertank auf.

Die Offenbarung umfasst auch eine Fördereinrichtung zur Förderung von lebenden Insekten, insbesondere von lebenden Insektenlarven, oder von lebenden Würmern, insbesondere in einer Reinigungsanlage wie oben beschrieben, wobei die Fördereinrichtung als Kontaktlospumpe ausgebildet ist, wobei die Kontaktlospumpe insbesondere eine Peristaltikpumpe ist.

Eine Larvenseparierungseinrichtung zur Separierung von lebenden Insekten, insbesondere von lebenden Insektenlarven, oder von lebenden Würmern von Wasser, insbesondere in einer Reinigungsanlage wie oben beschrieben, gemäß der vorliegenden Offenbarung weist eine Siebtrommel mit einem Mantelabschnitt, der Maschen mit einer Maschenbreite von 1,0 mm bis 2,5 mm, vorzugsweise von 1,3 mm bis 2,0 mm, und mit einerTransportschraube innerhalb der Siebtrommel zum Transportieren der Insekten oder der Würmer von einem Eintrittsende der Siebtrommel bis zu einem Austrittsende der Siebtrommel auf.

Verschiedene Ausführungsformen können ferner folgende Merkmale umfassen.

Der Mantelabschnitt der Siebtrommel ist bevorzugt ein Keildraht-Sieb. Die Siebtrommel weist vorzugsweise einen Innendurchmesser von 500 mm bis 3000 mm, bevorzugt 1000 mm bis 1500 mm, besonders bevorzugt von 1100 mm bis 1250 mm, auf.

Die Transportschraube weist bevorzugt eine Schraubenlänge auf, die sich über die gesamte Länge der Siebtrommel erstreckt. Die Transportschraube weist bevorzugt eine Schraubenhöhe von 5 mm bis 300 mm, vorzugsweise 50 mm bis 150 mm, besonders bevorzugt von 70 mm bis 100 mm, auf. Die Transportschraube weist bevorzugt eine Steigung von 200 mm bis 600 mm, bevorzugt 300 mm bis 400 mm, besonders bevorzugt von 330 mm bis 375 mm, auf.

Bevorzugt ist zumindest ein Spraybalken zum Sprühen einer Flüssigkeit mittels mit vorzugsweise mehreren Düsen vorgesehen. Der zumindest eine Spraybalken weist bevorzugt 5 bis 40 Düsen, vorzugsweise 8 bis 28 Düsen, auf. Bevorzugt ist der zumindest eine Spraybalken innerhalb der Siebtrommel angeordnet und/oder außerhalb der Siebtrommel angeordnet.

Bevorzugt ist zumindest ein Reinigungselement zur Reinigung des Mantelabschnitts der Siebtrommel vorgesehen. Bevorzugt ist das zumindest eine Reinigungselement zur Reinigung der Aussenseite der Siebtrommel ausgebildet und/oder als Bürstenelement ausgebildet.

Das zumindest eine Reinigungselement erstreckt sich vorzugsweise über die gesamte Länge der Siebtrommel.

Ein Verfahren zur Separierung von lebenden Insekten, insbesondere von lebenden Insektenlarven, oder von lebenden Würmern von Wasser mittels einer Larvenseparierungseinrichtung wie oben beschrieben, gemäß der vorliegenden Offenbarung umfasst die Schritte:
- Zuführen einer Mischung aus Insekten, insbesondere Insektenlarven, oder Würmern und Wasser, wobei die Mischung eine Temperatur von 5 °C bis 30 °C, vorzugsweise von 8 °C bis 15 °C oder 20 °C bis 26 °C aufweist, wobei das Zuführen vorzugsweise kontinuierlich erfolgt,
- Drehen der Siebtrommel, wobei die Mischung entwässert wird und die Insekten oder die Würmer zur Austrittsöffnung transportiert werden.

Verschiedene Ausführungsformen können ferner folgende Merkmale umfassen.

Die Siebtrommel weist bevorzugt eine Drehzahl von 2 bis 60 Umdrehungen pro Minute, vorzugsweise 5 bis 20 Umdrehungen pro Minute, besonders bevorzugt eine Drehzahl im Bereich von 10 bis 15 Umdrehungen pro Minute, auf.

Bevorzugt werden die lebenden Insekten oder die lebenden Würmer während dem Transport in der Siebtrommel mit Wasser aus dem zumindest einen Sprühbalken, vorzugsweise von zumindest einem innenseitig von der Siebtrommel angeordneten Sprühbalken, besprüht, wobei das Besprühen vorzugsweise kontinuierlich erfolgt und/oder mit einem Wasserduck von 2 bar bis 10 bar, vorzugsweise von 3 bar bis 7 bar. Zur Reinigung der Siebtrommel wird diese bevorzugt mit dem zumindest einen Sprühbalken, vorzugsweise mit einem außenseitig von der Siebtrommel angeordneten Sprühbalken, während der Rotation der Siebtrommel mit einer Flüssigkeit besprüht. Bevorzugt wird zur Reinigung der Siebtrommel diese von dem zumindest einen Reinigungselement während der Rotation der Siebtrommel kontaktiert.

Im Folgenden wird die Erfindung anhand von Ausführungsbeispielen und Zeichnungen näher erläutert. Dabei zeigt
Figur 1a: eine schematische Darstellung eines Ausführungsbeispiels einer erfindungsgemäßen Reinigungsanlage mit einem Reinigungsschritt;
Figur 1b: eine schematische Darstellung eines Ausführungsbeispiels einer erfindungsgemäßen Reinigungsanlage mit einem Reinigungsschritt und der Lagerung von Insekten oder Würmern;
Figur 2a: eine schematische Darstellung eines Ausführungsbeispiels einer erfindungsgemäßen Reinigungsanlage mit zwei Reinigungsschritten;
Figur 2b: eine schematische Darstellung eines Ausführungsbeispiels einer erfindungsgemäßen Reinigungsanlage mit zwei Reinigungsschritten und der Lagerung von Insekten oder Würmern;
Figur 2c: eine schematische Darstellung eines Ausführungsbeispiels einer erfindungsgemäßen Reinigungsanlage mit zwei Reinigungsschritten und der Lagerung von Insekten oder Würmern mit Verhältnis- bzw. Konzentrationskontrolle im Lagertank;
Figur 3: eine erfindungsgemäße Fördereinrichtung in einer Schnittdarstellung; und
Figuren 4a) und 4b): schematische Darstellungen einer erfindungsgemäßen Larvenseparierungseinrichtung.

Im Folgenden sind gleichlautende Elemente, auch wenn sie unterschiedliche Bezugszeichen tragen sollten, ähnlich oder identisch im Aufbau, sofern nichts anderes spezifiziert wird. Gleiches gilt für Bezugszeichen. Alle Ausführungsformen sind gleichermaßen für Insekten, insbesondere Insektenlarven, und Würmer einsetzbar.

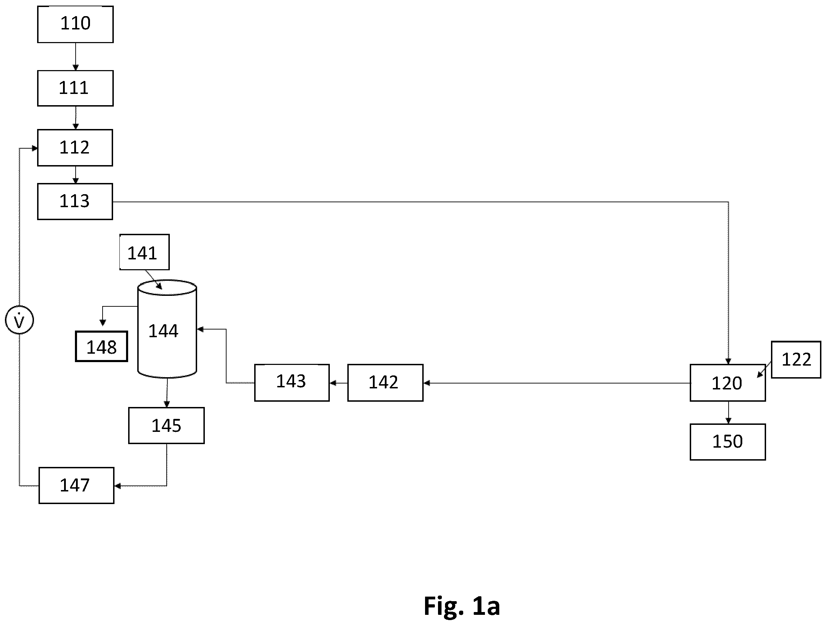

Fig. 1a zeigt ein beispielhaftes System gemäß der vorliegenden Offenbarung. Insbesondere wird ein System zur Behandlung von lebenden Insekten, insbesondere lebenden Larven, oder lebenden Würmern, mit einem Reinigungsschritt gezeigt. Das System kann auch als Reinigungsanlage bezeichnet werden. Innerhalb des Systems werden lebende Insekten oder Würmer mit einem geschlossenen Wasserkreislauf behandelt, der aus einer Insektenwaschvorrichtung oder Insektenseparierungsanlage 120, einer Reinigungseinrichtung bzw. Filtereinrichtung für Abwasser 142 und einer Wassertemperierungseinrichtung 145 besteht. Das Wasser dient dabei sowohl dem Transport als auch der Reinigung der Insekten und kann auch zur Reinigung bestimmter Elemente der Anlage, beispielsweise der Kisten, verwendet werden. Anstelle von Wasser kann auch eine andere geeignete Flüssigkeit verwendet werden, die es erlaubt, die Insekten gemeinsam mit der Flüssigkeit innerhalb des Systems zu bewegen. Eine Steuerung ist vorgesehen, um die Behandlung der Larven und beispielsweise die Verhältnisse zwischen Wasser und Larven im System überwachen und einstellen zu können.

Die Insekten, insbesondere lebenden Insekten, Larven oder Würmer, kommen aus der Züchtung 110 auf eine Wiegeeinrichtung 111. Die Wiegeeinrichtung 111 kann separat vorgesehen sein, oder in ein Transportmittel wie beispielsweise ein Förderband oder Transportgurt integriert sein. Es können auch Kisten, in denen die Insekten gezüchtet werden, vollständig gewogen werden. In der Wiegeeinrichtung 111 werden die geernteten Insekten (direkt oder indirekt mitsamt Kiste) gewogen und bestimmt, wie viel Wasser hinzugegeben werden muss um ein gewünschtes Gewichtsverhältnis von Larven zu Wasser, beispielsweise von etwa 1:10, zu erhalten. Hierfür werden die Insekten in eine Sammeleinrichtung 112 gebracht, zu der mittels einer Pumpe Frischwasser hinzugefügt wird, bis das gewünschte Gewichtsverhältnis erreicht wird. Alternativ oder zusätzlich kann die Wiegeeinrichtung 111 auch in der Sammeleinrichtung 112 integriert vorgesehen sein oder die Sammeleinrichtung 112 kann auf einer Wiegeeinrichtung 111, bspw. einer Wägezelle, angeordnet sein. Das Verhältnis 1:10 ist hier lediglich beispielhaft genannt und es kann je nach Insektenart und/oder Anlagenspezifikationen eine andere Wassermenge beigegeben werden. Als vorteilhaft haben sich Mischverhältnisse zwischen Larven und Wasser von 1:1 bis 1:10 erwiesen. Das Wasser kann vorher mittels eines Wärmetauschers 145 temperiert werden. Durch gekühltes Wasser ist es möglich, die Insekten oder Würmer zu kühlen und in die Diapause oder Dormanz zu bringen. Dadurch werden die Insekten metabolisch deaktiviert und können besser gelagert werden. Dies wird bei Temperaturen unter 15 °C, bevorzugt 8 °C bis 15 °C, erreicht. Wärmeres Wasser, also gewärmtes Wasser oder Wasser mit Umgebungstemperatur von bspw. 20 °C bis 30 °C, vorzugsweise bis 26 °C, kann abhängig von der Verschmutzung zu besseren Reinigungsergebnissen führen. Des Weiteren wird den Energieverbrauch reduziert, da das Wasser nicht gekühlt oder gewärmt werden muss. Allerdings können die Insekten oder Würmer weniger lang gelagert werden, da sie nicht in die Diapause versetzt werden. Die Mischung aus Insekten, Larven oder Würmern mit Wasser wird dann mittels einer ersten Fördereinrichtung 113 zu einer Larvenseparierungseinrichtung 120 gepumpt. Diese separiert Wasser von Larven, um das Verhältnis von Larven zu Wasser in gewünschter Weise zu verändern und wird später beispielhaft genauer beschrieben. Dieser Vorgang kann auch als Entwässern bezeichnet werden. Des Weiteren werden die Insekten bei diesem Vorgang gewaschen und Ausscheidungen sowie Futterreste können entfernt werden. Die Larvenseparierungseinrichtung 120 kann auch als Reinigungseinrichtung bezeichnet werden. Das Abwasser wird einem Substratfilter 142 zugeführt, der Feststoffe aus dem Wasser filtert, und mittels einer Pumpe 143 in einen Wassertank 144 gepumpt. Anstelle eines Filters 142 kann auch eine Zentrifuge verwendet werden.

Um das Gewichtsverhältnis nach der Larvenseparierungseinrichtung 120 einstellen zu können, das Wasser, in welchem die Insekten oder Würmer gelagert waren, auszutauschen, oder ein besseres Reinigungsergebnis zu erzielen, ist eine Frischwasserzufuhr 122 vorgesehen. Die Menge an zugeführtem Frischwasser kann durch einen Durchflussmesser (nicht gezeigt) überprüft werden. Insbesondere kann die Frischwassermenge volumenbasiert oder gewichtsbasiert bestimmt werden. Das Wasser wird rezirkuliert, indem das Wasser mittels des Substratfilters 142 gereinigt wird und durch die Pumpe 143 in den Wassertank 144 gepumpt wird. Dort kann Abwasser entfernt werden und gegebenenfalls durch eine Frischwasserzufuhr frisches Wasser hinzugefügt werden. Die Zuführung von Frischwasser und die Entnahme von Abwasser kann auch an anderen Stellen im System zweckdienlich sein.

Wie oben beschrieben wird das gereinigte Wasser aus dem Wassertank 144 mittels eines Wärmetauschers 145 wie oben beschrieben temperiert und dem Kreislauf wieder zugeführt, indem es durch eine Pumpe 147 in die Sammeleinrichtung 112 gefördert und dort mit den Insekten oder Würmern vermischt wird. Es kann auch ein Durchflussmesser V vorgesehen sein, um die zugeführte Wassermenge zu messen. Die zugegebene Wassermenge kann auch gewichtsbasiert kontrolliert werden. Ferner kann der Wärmetauscher 145 auch mit Heizelementen ausgestattet sein, um das Wasser zu temperieren. Ferner können auch die Sammeleinrichtung 112, die Fördereinrichtung 113, 121 und/oder die Separierungseinrichtung 120 temperiert sein, um die Wassertemperatur einstellen zu können.

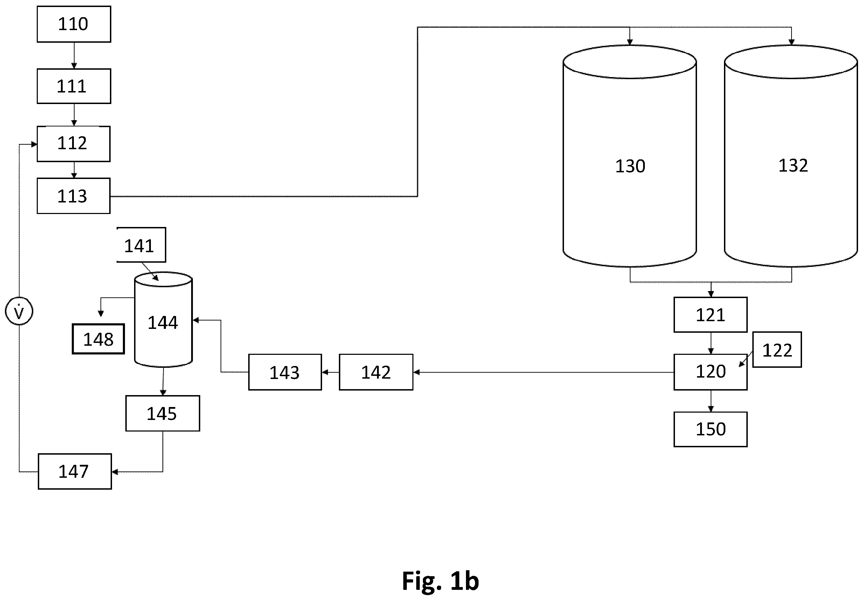

Fig. 1b zeigt ein beispielhaftes System gemäß der vorliegenden Offenbarung. Das System weist zusätzlich zu der in Fig. 1a gezeigten Ausführungsform noch die Möglichkeit der Lagerung von Insekten oder Würmern auf. Innerhalb des Systems werden lebende Insekten oder Würmer mit einem geschlossenen Wasserkreislauf behandelt, der aus mindestens einem Speichertank 130, 132, einer Insektenwaschvorrichtung oder Insektenseparierungsanlage 120, einer Reinigungseinrichtung für Abwasser 142 und einer Wassertemperierungseinrichtung 145 besteht. Das Wasser dient dabei sowohl dem Transport als auch der Reinigung der Insekten. Anstelle von Wasser kann auch eine andere geeignete Flüssigkeit verwendet werden, die es erlaubt, die Insekten gemeinsam mit der Flüssigkeit innerhalb des Systems zu bewegen. Eine Steuerung ist vorgesehen, um die Behandlung der Larven und beispielsweise die Verhältnisse zwischen Wasser und Larven im System überwachen und einstellen zu können.

Die Insekten, insbesondere lebende Insekten, Larven oder Würmer, kommen aus der Züchtung 110 auf eine Wiegeeinrichtung 111. Die Wiegeeinrichtung 111 kann separat vorgesehen sein, oder in ein Transportmittel wie beispielsweise ein Förderband oder Transportgurt integriert sein. Es können auch Kisten, in denen die Insekten gezüchtet werden, vollständig gewogen werden. In der Wiegeeinrichtung 111 werden die geernteten Insekten (direkt oder indirekt mitsamt Kiste) gewogen und bestimmt, wie viel Wasser hinzugegeben werden muss um ein gewünschtes Gewichtsverhältnis von Larven zu Wasser, beispielsweise von etwa 1:10, zu erhalten. Hierfür werden die Insekten in eine Sammeleinrichtung 112 gebracht, zu dem mittels einer Pumpe Frischwasser hinzugefügt wird, bis das gewünschte Gewichtsverhältnis erreicht wird. Alternativ oder zusätzlich kann die Wiegeeinrichtung 111 auch in der Sammeleinrichtung 112 vorgesehen sein oder die Sammeleinrichtung 112 kann auf einer Wiegeeinrichtung 111, bspw. einer Wägezelle, angeordnet sein. Das Verhältnis 1:10 ist hier lediglich beispielhaft genannt und es kann je nach Insektenart und/oder Anlagenspezifikationen eine andere Wassermenge beigegeben werden. Als vorteilhaft haben sich Mischverhältnisse zwischen Larven und Wasser von 1:1 bis 1:10 erwiesen. Das Wasser wird vorher mittels eines Wärmetauschers 145 temperiert. Durch gekühltes Wasser ist es möglich, die Insekten oder Würmer zu kühlen und in die Diapause oder Dormanz zu bringen. Dadurch werden die Insekten metabolisch deaktiviert und können besser gelagert werden. Dies wird bei Temperaturen unter 15 °C, bevorzugt 8 °C bis 15 °C, erreicht. Wärmeres Wasser, also gewärmtes Wasser oder Wasser mit Umgebungstemperatur von bspw. 20 °C bis 30 °C, vorzugsweise bis 26 °C, kann abhängig von der Verschmutzung zu besseren Reinigungsergebnissen führen. Des Weiteren wird den Energieverbrauch reduziert, da das Wasser nicht gekühlt oder gewärmt werden muss. Allerdings können die Insekten oder Würmer weniger lang gelagert werden, da sie nicht in die Diapause versetzt werden. Die Mischung aus Insekten und Larven oder Würmern wird dann mittels einer ersten Fördereinrichtung 113 in den mindestens einen Speichertank 130, 132 transportiert. Die erste Fördereinrichtung 113 kann dabei insbesondere als Kontaktlospumpe, beispielsweise als Peristaltikpumpe, ausgebildet sein, um die Insekten nicht zu verletzen oder zu quetschen. In den Speichertanks 130, 132 können die metabolisch deaktivierten, d. h. in der Diapause befindlichen, Insekten bis zur weiteren Verarbeitung gelagert werden.

Die gereinigten Insekten werden mittels einer zweiten Fördereinrichtung 121, die ebenfalls bevorzugt als Kontaktlospumpe ausgebildet ist, aus dem einen oder den mehreren Speichertanks 130, 132 gepumpt. Das Verhältnis von Insekten zu Wasser beträgt hier zwischen 1:1 und 1:10, bevorzugt 1:4.

Die Larvenseparierungseinrichtung 120, die der zweiten Fördereinrichtung 121 nachgelagert ist und später beispielhaft genauer beschrieben wird, separiert Wasser von Larven, um das Verhältnis von Larven zu Wasser in gewünschter Weise zu verändern. Dies kann auch als Entwässern bezeichnet werden. Des Weiteren werden die Insekten bei diesem Vorgang gewaschen und Ausscheidungen sowie Futterreste können entfernt werden. Die Larvenseparierungseinrichtung 120 kann auch als Reinigungseinrichtung bezeichnet werden. Das Abwasser wird einem Substratfilter 142 zugeführt, der Feststoffe aus dem Wasser filtert, und mittels einer Pumpe 143 in einen Wassertank 144 gepumpt. Anstelle eines Filters 142 kann auch eine Zentrifuge verwendet werden.

Um das Gewichtsverhältnis nach der Larvenseparierungseinrichtung 120 einstellen zu können, das Wasser, in welchem die Insekten oder Würmer gelagert waren, auszutauschen, oder ein besseres Reinigungsergebnis zu erzielen, ist eine Frischwasserzufuhr 122 vorgesehen. Die Menge an zugeführtem Frischwasser kann durch einen Durchflussmesser (nicht gezeigt) überprüft werden. Insbesondere kann die Frischwassermenge volumenbasiert oder gewichtsbasiert bestimmt werden. Das Wasser wird rezirkuliert, indem das Wasser mittels des Substratfilters 142 gereinigt wird und durch die Pumpe 143 in den Wassertank 144 gepumpt wird. Dort kann Abwasser entfernt werden und gegebenenfalls durch eine Frischwasserzufuhr frisches Wasser hinzugefügt werden. Die Zuführung von Frischwasser und die Entnahme von Abwasser kann auch an anderen Stellen im System zweckdienlich sein.

Wie oben beschrieben wird das gereinigte Wasser aus dem Wassertank 144 mittels eines Wärmetauschers 145 wie oben beschrieben temperiert und dem Kreislauf wieder zugeführt, indem es durch eine Pumpe 147 in den Sammelbehälter 112 gefördert und dort mit den Insekten oder Würmern vermischt wird. Es kann auch ein Durchflussmesser V vorgesehen sein, um die zugeführte Wassermenge zu messen. Die zugegebene Wassermenge kann auch gewichtsbasiert kontrolliert werden. Ferner kann der Wärmetauscher 145 auch mit Heizelementen ausgestattet sein, um das Wasser zu temperieren. Ferner können auch die Sammeleinrichtung 112, die Fördereinrichtung 113, 121 und/oder die Separierungseinrichtung 120 temperiert sein, um die Wassertemperatur einstellen zu können.

Somit wird ein effizienter Wasserkreislauf gewährleistet, der Insekten schonend transportiert, wäscht und lagert und gleichzeitig die anfallende Abwassermenge reduziert.

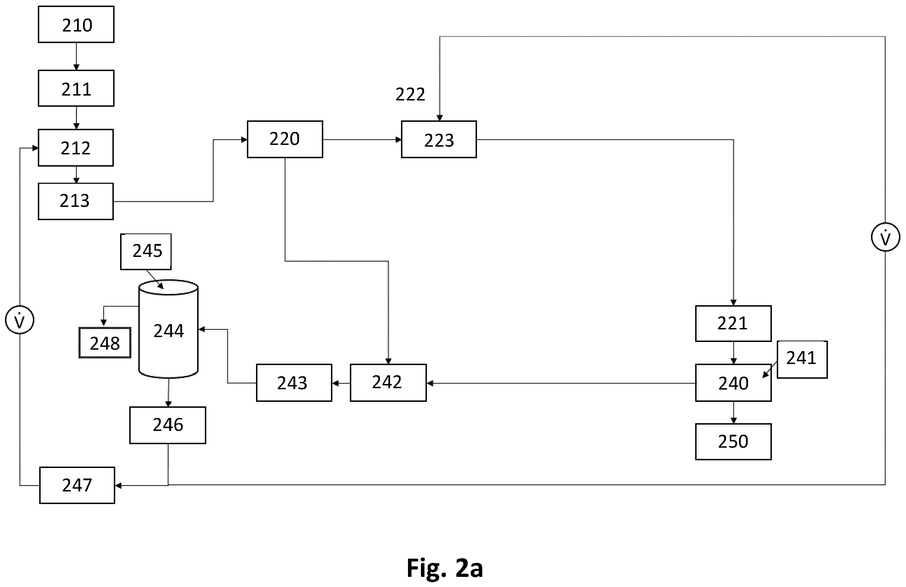

Fig. 2a zeigt ein Beispiel einer weiteren Ausführungsform einer Reinigungsanlage gemäß der vorliegenden Offenbarung mit zwei Reinigungsschritten. Sofern nicht anders gekennzeichnet, entsprechen gleichnamige Elemente den Elementen des Beispiels aus Fig. 1a und 1b und können in beiden Ausführungsformen verwendet werden.

Die aus der Züchtung 210 kommenden lebenden Insekten, insbesondere lebende Larven oder lebende Würmer, werden in der Wiegeeinrichtung 211 gewogen und in den Sammelbehälter 212 transportiert, in dem sie mit Wasser gemischt werden, das aus dem Wassertank 244 mittels der Pumpe 247 gepumpt wird und mittels des Wärmetauschers 246 temperiert wird. Die Wassertemperaturen können wie im Zusammenhang mit Fig. 1a und 1b beschrieben gewählt werden. Das Wiegen kann auch im Sammelbehälter bzw. der Sammeleinrichtung 212 durchgeführt werden oder die Sammeleinrichtung kann auf einer Wägezelle positioniert sein. Der Wärmetauscher 246 kann das Wasser kühlen oder mit Heizelementen ausgestattet sein, um das Wasser zu temperieren. Es kann auch zweckdienlich sein, Wasser bei Umgebungstemperatur zu verwenden.

Auch hier beträgt das Gewichtsverhältnis von Insekten zu Wasser vorzugsweise 1:10, es können aber auch andere Gewichtsverhältnisse zweckdienlich sein. Die Mischung wird von einer Pumpe 213, vorzugsweise einer Kontaktlospumpe wie beispielsweise einer Peristaltikpumpe, zur ersten Larvenseparierungseinrichtung 220 gepumpt. Dort werden die Insekten vom Wasser bis zu einem gewünschten Gewichtsverhältnis separiert, mit anderen Worten entwässert, wobei das überschüssige Wasser in den Substratfilter 242 fließt, gereinigt wird und durch die Pumpe 243 in den Wassertank 244 gepumpt und dort gelagert wird. Die gereinigten Insekten oder Würmer oder die gereinigte Mischung aus Wasser und Insekten oder Würmern wird aus der Larvenseparierungseinrichtung 220 in eine weitere Sammeleinrichtung 223 transportiert. Das Gewichtsverhältnis von Insekten oder Würmern zu Wasser kann in der Sammeleinrichtung 223 zwischen 1:1 und 1:10, vorzugsweise 1:4, betragen und durch die Frischwasserzufuhr 222 eingestellt werden. Auch hier kann ein Durchflussmesser V oder ein gewichtsbasierter Sensor vorgesehen sein, um die Frischwassermenge zu messen.

Die Insekten-Wassermischung wird dann mittels der zweiten Fördereinrichtung 221, die ebenfalls bevorzugt als Kontaktlospumpe ausgebildet ist, zu einer zweiten Larvenseparierungseinrichtung 240 gebracht, wo Insekten oder Würmer und Wasser, gegebenenfalls unter Zufuhr von Frischwasser 241, teilweise oder vollständig voneinander getrennt werden. Die Larvenseparierungseinrichtung 240 kann ähnlich oder identisch zur Larvenseparierungseinrichtung 220 sein. Das abgeschiedene Wasser aus der zweiten Larvenseparierungseinrichtung 240 wird im Substratfilter 242 gereinigt und von der Pumpe 243 in den Wassertank 244 gepumpt. Dort kann je nach Zustand des Wassers Abwasser 248 abgepumpt werden und Frischwasser 245 zugeführt werden.

Wie oben bereits beschrieben und wie im Beispiel gemäß Fig. 1 angegeben, wird das Wasser aus dem Wassertank 244 rezirkuliert und im Sammelbehälter 212 mit Insekten gemischt.

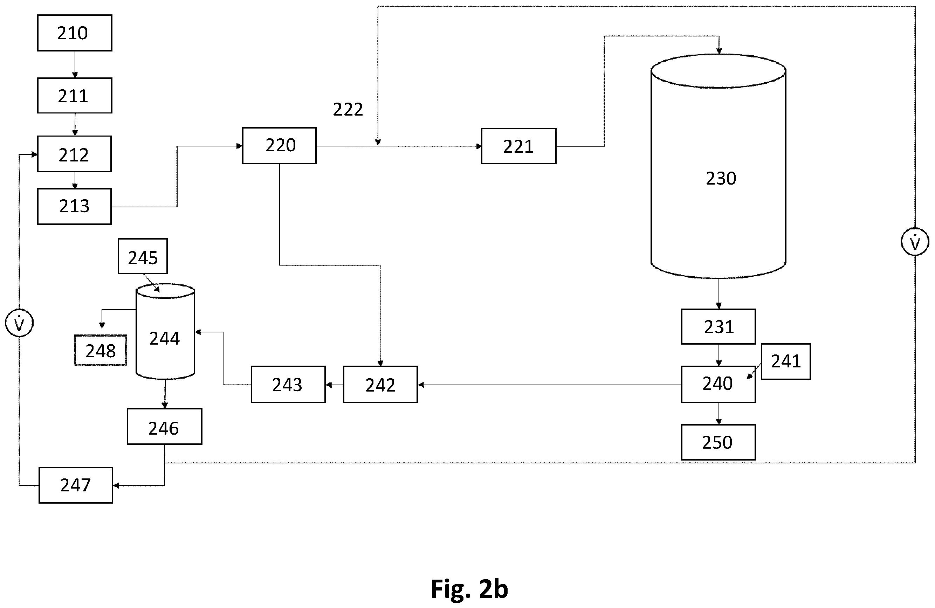

Fig. 2b zeigt ein Beispiel einer weiteren Ausführungsform einer Reinigungsanlage gemäß der vorliegenden Offenbarung mit zwei Reinigungsschritten und der Möglichkeit zur Lagerung der Insekten oder Würmer. Sofern nicht anders gekennzeichnet, entsprechen gleichnamige Elemente den Elementen des Beispiels aus Fig. 1a und 1b und 2a und können in beiden Ausführungsformen verwendet werden.

Die Ausführungsform entspricht bis zur ersten Larvenseparierungseinrichtung 220 der in Fig. 2a gezeigten. In der ersten Larvenseparierungseinrichtung 220 werden die Insekten vom Wasser bis zu einem gewünschten Gewichtsverhältnis separiert, mit anderen Worten entwässert, wobei das überschüssige Wasser in den Substratfilter 242 fließt, gereinigt wird und durch die Pumpe 243 in den Wassertank 244 gepumpt und dort gelagert wird. Wie oben beschrieben kann das Gewichtsverhältnis von Insekten zu Wasser nach der ersten Larvenseparierungseinrichtung 220 zwischen 1:1 und 1:10, vorzugsweise 1:4, betragen und durch die Frischwasserzufuhr 222 eingestellt werden. Auch hier kann ein Durchflussmesser V oder ein gewichtsbasierter Sensor vorgesehen sein, um die Frischwassermenge zu messen.

Die Insekten-Wassermischung wird dann mittels der zweiten Fördereinrichtung 221, die ebenfalls bevorzugt als Kontaktlospumpe ausgebildet ist, in einen Speichertank 230 gepumpt. Dort kann je nach Bedarf weiteres Frischwasser zugeführt werden.

Wenn die Insekten der weiteren Verarbeitung 250 zugeführt werden sollen, werden sie mittels einer dritten Fördereinrichtung 231 aus dem Speichertank 230 zu einer zweiten Larvenseparierungseinrichtung 240 gebracht, wo Insekten und Wasser, gegebenenfalls unter Zufuhr von Frischwasser 241, teilweise oder vollständig voneinander getrennt werden. Die Larvenseparierungseinrichtung 240 ist ähnlich oder identisch zur Larvenseparierungseinrichtung 220. Das abgeschiedene Wasser aus der zweiten Larvenseparierungseinrichtung 240 wird im Substratfilter 242 gereinigt und von der Pumpe 243 in den Wassertank 244 gepumpt. Dort kann je nach Zustand des Wassers Abwasser 248 abgepumpt werden und Frischwasser 245 zugeführt werden.

Wie oben bereits beschrieben und wie im Beispiel gemäß Fig. 1a und 1b und 2a angegeben, wird das Wasser aus dem Wassertank 244 rezirkuliert und im Sammelbehälter 212 mit Insekten gemischt.

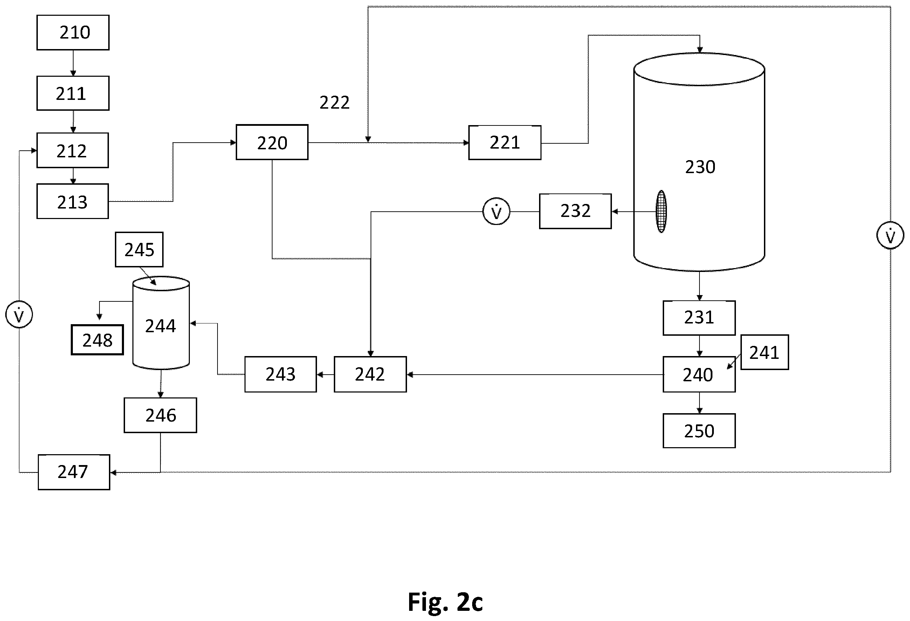

Fig. 2c zeigt ein Beispiel einer weiteren Ausführungsform einer Reinigungsanlage gemäß der vorliegenden Offenbarung. Diese Ausführungsform bietet die Möglichkeit, das Verhältnis von Wasser zu Larven bzw. Würmern im Tank einzustellen und so die Larven- oder Würmermenge im Speichertank flexibel zu gestalten. Die Ausführungsform ist weitestgehend identisch mit der in Fig. 2b gezeigten. Im Gegensatz zu dem in Fig. 2b gezeigten Beispiel ist hier eine Wasserpumpe 232 mit nachgelagertem Durchflussmesser V oder einem gewichtsbasierten Sensor vorgesehen, die Wasser abpumpen und dadurch das Gewichtsverhältnis zwischen Insekten und Wasser im Speichertank 230 steuern kann. Vorzugsweise ist ein Sieb oder eine Rückhaltevorrichtung eingebaut, die verhindert, dass Insekten mit dem Wasser durch die Pumpe 232 abgepumpt werden.

Durch das unter Bezugnahme auf die oben beschriebenen Figuren vorgestellte System werden die Züchtung und die weitere Verarbeitung der Insekten prozessual voneinander getrennt, wodurch eine höhere Flexibilität in der Produktion erreicht werden kann. Ferner können die Insekten transportiert, gewaschen und gelagert werden, ohne sie zu verletzen.

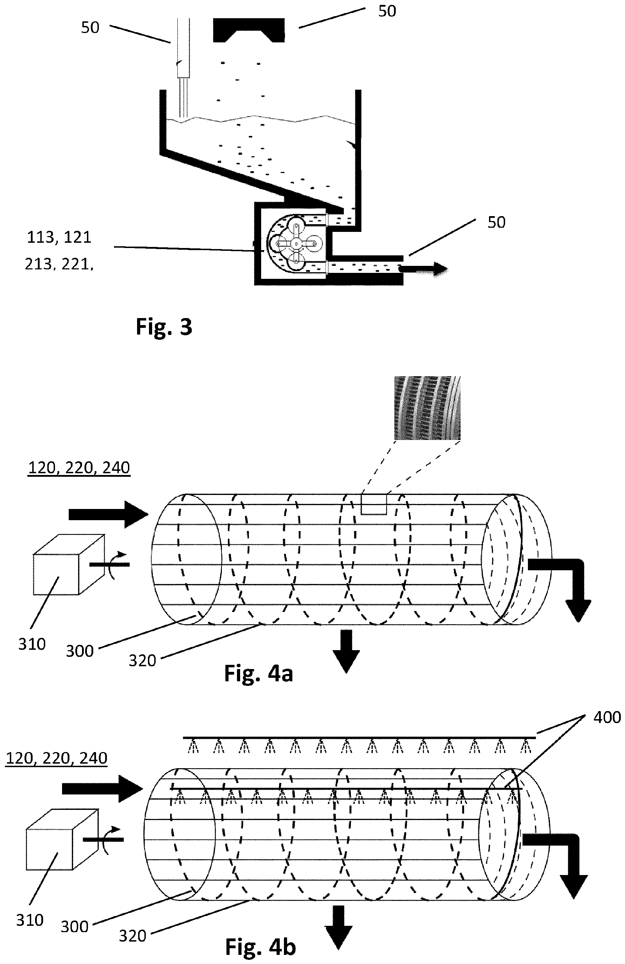

Fig. 3 zeigt eine Fördereinrichtung 113, 121, 213, 221, 231, die zur Förderung eines Gemisches aus Insekten und Wasser wie oben beschrieben geeignet ist. In Fig. 3 wird eine Situation dargestellt, in der Insekten durch eine Larvenzufuhr 500 mit Wasser, welches aus einer Wasserzufuhr 501 gespeist wird, gemischt werden und das Gemisch durch eine Fördereinrichtung, insbesondere eine Kontaktlospumpe oder Peristaltikpumpe, zu einem Auslass 502 befördert werden. Anstelle von Wasser kann auch eine andere geeignete nichtviskose Flüssigkeit verwendet werden, die es erlaubt, die Mischung aus Insekten und Flüssigkeit zu pumpen. Die vorgeschlagene Peristaltikpumpe ist eine Art kontaktloser Pumpe, bei der die Rotorblätter die zu pumpende Flüssigkeit nicht direkt berühren.

Alternativ können in allen Ausführungsformen der vorliegenden Offenbarung andere schonende Fördereinrichtungen wie bspw. Schraubenspindelpumpen, Drehkolbenpumpen, Kreiskolbenpumpen, Kolbenpumpen, Exzenterschneckenpumpen oder langsam laufende Kreiselpumpen eingesetzt werden.

Bevorzugt beträgt das Gewichtsverhältnis von Insekten zu Flüssigkeit zwischen 1:10 und 1:1. Die Pumpkapazität kann zwischen 5 und 100 m³/h betragen. Die Größe der zu pumpenden Insekten ist bevorzugt kleiner als 35 mm.

Die Fördereinrichtung kann insbesondere in einem System wie in Fig. 1 und 2 gezeigt eingesetzt werden. Dadurch können lebende Insekten sicher und effizient transportiert werden, ohne diese zu verletzen. Ferner wird kein unnötiger Stress auf die Tiere ausgeübt. Die Reduktion von Stress kann auch durch das Kühlen der Flüssigkeit auf Temperaturen unter 15 °C, bevorzugt 8 °C bis 15 °C, erreicht werden, wodurch die Insekten in die Diapause versetzt werden.

Die Figuren 4a und 4b zeigen eine beispielhafte Larvenseparierungseinrichtung 120, 220, 240, die sich nur durch den oder die zusätzlichen Spraybalken 400 unterscheiden. Die Larvenseparierungseinrichtung 120, 220, 240 kann insbesondere als Siebtrommel und bevorzugt als Keildrahtsieb ausgebildet sein. Die Larvenseparierungseinrichtung 120, 220, 240 wird durch einen Antrieb 310 angetrieben, insbesondere in Rotation versetzt. Ferner ist im Inneren der Trommel eine Transportschraube 300 angeordnet. Die Larvenseparierungseinrichtung 120, 220, 240 weist eine Einlassseite und eine Auslassseite auf (gekennzeichnet durch die Pfeile in den Figuren 4a und 4b), wobei die Insekten mittels der im Inneren angebrachten Transportschraube 300 schonend von der Einlass- zur Auslassseite transportiert werden. Dabei erstreckt sich die Transportschraube über die gesamte Länge der Siebtrommel und weist eine Schraubenhöhe von 5 mm bis 300 mm, vorzugsweise 50 mm bis 150 mm, besonders bevorzugt von 70 mm bis 100 mm auf. Die Steigung beträgt dabei zwischen 200 mm und 600 mm, bevorzugt 300 mm und 400 mm, besonders bevorzugt 330 mm und 375 mm. Dadurch kann einerseits überschüssiges Wasser oder ein gewünschter Anteil an Wasser entfernt werden, und andererseits können die Insekten gewaschen werden, ohne sie zu verletzen oder großen Stress auf sie auszuüben.

Zusätzlich können wie in Fig. 4b gezeigt Spraybalken 400 vorgesehen sein, die aus mehreren Düsen innerhalb der Larvenseparierungseinrichtung 120, 220, 240 Wasser versprühen und/oder außerhalb der Larvenseparierungseinrichtung 120, 220, 240 Wasser versprühen. Durch die zusätzliche Wasserzufuhr im Inneren der Larvenseparierungseinrichtung 120, 220, 240 kann der Waschvorgang verbessert werden, während durch die Wasserzufuhr von außen die Siebtrommel gereinigt werden kann. Es kann auch eine andere Flüssigkeit als Wasser oder ein Gemisch aus Wasser und einer weiteren Flüssigkeit versprüht werden. Jeder der Spraybalken 400 weist 5 bis 40 Düsen, bevorzugt 8 bis 28 Düsen für den Spraybalken 400 im Inneren und 5 bis 40 Düsen für den Spraybalken 400 außerhalb der Siebtrommel auf. Der Wasserdruck kann zwischen 2 bar und 10 bar, bevorzugt zwischen 3 bar und 7 bar betragen.

Alternativ oder zusätzlich kann außen an der Siebtrommel ein Reinigungselement vorgesehen sein, dass beispielsweise als Bürste ausgebildet ist.

Bevorzugt ist die Geometrie der Larvenseparierungseinrichtung 120, 220, 240 bzw. der Siebtrommel auf die Beförderung und das Waschen von Insekten abgestimmt. Dadurch werden Verletzungen der Insekten, Stress und das Verstopfen des Siebs verhindert und die Reinigung des Siebs von Resten aus dem Züchtungsvorgang wird vereinfacht.

Bevorzugt weist die Larvenseparierungseinrichtung 120, 220, 240 eine Siebtrommel mit einer Mantelfläche 320 auf, die Maschen mit einer Maschenbreite von 1,0 bis 2,5 mm, bevorzugt von 1,3 bis 2,0 mm aufweist. Insbesondere kann die Mantelfläche 320 des Siebs eine Maschengröße von 20 bis 70 %, vorzugsweise von 30 bis 50 %, des Larvendurchmessers aufweisen, wobei von einer Larvengröße von 3 bis 5 mm im Durchmesser und 12 bis 20 mm in der Länge ausgegangen wird. Derartige Maschen stellen Löcher bzw. Öffnungen in der Mantelfläche der Siebtrommel dar. In Fig. 4a ist beispielhaft für die Figuren 4a und 4b eine Detailansicht der Mantelfläche 320 sowie der oben beschriebenen Maschen gezeigt.

Die Siebtrommel hat beispielsweise einen Innendurchmesser von 500 mm bis 3000 mm, bevorzugt 1000 mm bis 1500 mm, bevorzugt von 1100 mm bis 1250 mm. Die Siebtrommel ist in der Regel zylindrisch ausgeformt, kann jedoch auch konisch zulaufend in eine der beiden Richtungen sein.

Die Drehzahl kann 2 bis 60 Umdrehungen/min, bevorzugt 5 bis 20 Umdrehungen/min, besonders bevorzugt 10 bis 15 Umdrehungen/min betragen.

Die Larvenseparierungseinrichtung nach Fig. 4a oder 4b kann, auch in Kombination mit der Fördereinrichtung gemäß Fig. 3, in einem System oder einer Reinigungsanlage nach Fig. 1 oder 2 verwendet werden.

Die Sammeleinrichtungen gemäß den oben beschriebenen Beispielen können auch der Mischung von Insekten oder Larven mit einer Flüssigkeit, bspw. Wasser, dienen. Zudem kann die Sammeleinrichtung auch zur Lagerung der Insekten verwendet werden. Ferner kann die Sammeleinrichtung auch der Temperierung oder dem Wiegen dienen.

Da, falls mehrere Separierungseinrichtungen vorgesehen sind, der Verschmutzungsgrad des Wassers unterschiedlich sein kann, können auch mehrere unabhängige oder miteinander verbundene Wasserkreisläufe vorgesehen sein. Dies umfasst insbesondere mehrere Filteranlagen und/oder mehrere Wassertanks. Das Wasser kann auch zum Waschen der Kisten in der Anlage wiederverwendet werden.

Falls es zweckdienlich und möglich ist, kann eine oder mehrere der Fördereinrichtungen durch ein Gefälle ersetzt werden. Die Insekten oder Würmer werden demnach nicht aktiv gefördert, sondern durch die Schwerkraft dem nächsten Prozessschritt zugeführt.

Ferner kann eine Schnecke als kombinierte Förder- und Sammeleinrichtung vorgesehen sein. Diese kann Möglichkeiten zur Temperierung und zur Frischwasserzufuhr aufweisen.

Durch die Kühlung des Wassers auf unter 15 °C, bevorzugt 8 °C bis 15 °C, können die Insekten oder Würmer bereits während des Transports bzw. des Reinigungsvorgangs betäubt werden. Die Verwendung von Wasser bei Umgebungstemperatur, bspw. 15 °C bis 30 °C, insbesondere 20 °C bis 26 °C, kann je nach Verschmutzung ein besseres Reinigungsergebnis erzielen und ist energieeffizienter, da die Notwendigkeit der Kühlung entfällt. Dies ist jedoch möglicherweise auch mit mehr Stress für die Tiere verbunden, da diese während der Verarbeitung nicht oder nur teilweise betäubt sind. Ferner wird die Lagerdauer der Tiere verkürzt, da sie nicht in die Diapause versetzt werden.

Die Erfindung wurde anhand einer Reinigungsanlage mit einem Waschschritt, einer Reinigungsanlage mit einem Waschschritt und Lagerung, einer Reinigungsanlage mit zwei Waschschritten, einer Reinigungsanlage mit zwei Waschschritten und Lagerung sowie einer Reinigungsanlage mit zwei Waschschritten und Lagerung mit Verhältnis bzw. KonzentrationsKontrolle im Lagertank beschrieben. Es können jedoch auch mehr als zwei Wasch- bzw. Separierungsschritte oder weitere Zwischenlagerungsschritte vorgesehen sein.

Die Offenbarung umfasst ebenfalls ein Verfahren zur Reinigung von lebenden Insekten oder Würmern, bevorzugt mit einer Reinigungsanlage wie oben beschrieben.

Ferner umfasst die Offenbarung eine Fördereinrichtung zur Förderung von lebenden Insekten oder Würmern, die die Insekten oder Würmer gemeinsam mit einer Trägerflüssigkeit schonend pumpt, ohne die Insekten zu verletzen oder Stress auf sie auszuüben.

Des Weiteren werden eine Larvenseparierungseinrichtung zur Separierung von lebenden Insekten oder Würmern von Wasser sowie ein entsprechendes Verfahren bereitgestellt, wodurch lebende Insekten schonend transportiert und gewaschen werden können und weiterhin das Gewichtsverhältnis von Insekten zu Wasser eingestellt werden kann.

Gemäß der vorliegenden Offenbarung werden eine Reinigungsanlage zur Reinigung von lebenden Insekten oder Würmern sowie ein entsprechendes Verfahren zur Reinigung von lebenden Insekten oder Würmern bereitgestellt, die eine schonende und stressfreie Handlung bzw. Verarbeitung der Insekten oder Würmer gewährleistet. Die Flüssigkeit, die zum Transport und zur Reinigung der Insekten oder Würmer verwendet wird, wirkt als Puffer, wodurch weniger mechanische Kräfte auf die Tiere wirken. Ferner wird verhindert, dass die Tiere zerdrückt werden. Diese können somit stressfreier transportiert werden und eine gleichmäßige Qualität kann gewährleistet werden. Daher ist das Verhältnis von Insekten oder Würmern zu Transportflüssigkeit wichtig für die Prozessführung. Die Transportflüssigkeit wird gereinigt und vollständig oder zu großen Teilen rezirkuliert, wodurch ein geschlossener Wasserkreislauf entsteht. Dies führt zu einem erheblich geringeren Wasserverbrauch. Ferner werden die Insekten durch kühlende Flüssigkeit metabolisch deaktiviert und in die Diapause versetzt, wodurch sie in einem Speichertank gelagert werden können. Durch das vorgestellte System wird die Züchtung von der Reinigung und Lagerung getrennt, wodurch beide Bereiche unabhängig voneinander betrieben werden können und die Flexibilität in der Anlage erhöht wird.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang der folgenden Ansprüche zu verlassen. Insbesondere umfasst die Erfindung ebenfalls Ausführungsformen mit jeglicher Kombination von Merkmalen, die vorstehend zu verschiedenen Aspekten und/oder Ausführungsformen genannt oder gezeigt sind.

Die Erfindung umfasst ebenfalls einzelne Merkmale in den Figuren auch wenn sie dort im Zusammenhang mit anderen Merkmalen gezeigt sind und/oder vorstehend nicht genannt sind.

Im Weiteren schließt der Ausdruck "umfassen" und Ableitungen davon andere Elemente oder Schritte nicht aus.

Alle Bezugszeichen in den Ansprüchen sind nicht als den Umfang der Ansprüche einschränkend zu verstehen.

### Liste der Bezugszeichen

- 110: Ernten
- 111: Wiegeeinrichtung
- 112: Sammelbehälter/Sammeleinrichtung
- 113: Erste Fördereinrichtung
- 120: Erste Larvenseparierungseinrichtung
- 121: Zweite Fördereinrichtung
- 122: Frischwasserzufuhr
- 130: Speichertank
- 132: Speichertank
- 141: Frischwasserzufuhr
- 142: Filtereinrichtung/Substratfilter/Feststofffilter
- 143: Wasserpumpe
- 144: Wassertank
- 145: Wärmetauscher
- 147: Wasserpumpe
- 148: Abwasser
- 150: Verarbeitung
- 210: Ernten
- 211: Wiegeeinrichtung
- 212: Sammelbehälter/Sammeleinrichtung
- 213: Erste Fördereinrichtung
- 220: Erste Larvenseparierungseinrichtung
- 221: Zweite Fördereinrichtung
- 222: Frischwasserzufuhr
- 223: Sammelbehälter/Sammeleinrichtung
- 230: Speichertank
- 231: Dritte Fördereinrichtung
- 232: Wasserpumpe
- 240: Zweite Larvenseparierungseinrichtung
- 241: Frischwasserzufuhr
- 242: Filtereinrichtung/Substratfilter/Feststofffilter
- 243: Wasserpumpe
- 244: Wassertank
- 245: Frischwasserzufuhr
- 246: Wärmetauscher
- 247: Wasserpumpe
- 248: Abwasser
- 250: Verarbeitung
- 300: Transportschraube
- 310: Antrieb
- 320: Mantelfläche
- 400: Spraybalken
- 500: Larvenzufuhr
- 501: Wasserzufuhr
- 502: Auslass
- V: Durchflussmesser

## Patentansprüche

1. Reinigungsanlage zur Reinigung von lebenden Insekten, insbesondere von lebenden Insektenlarven, oder von lebenden Würmern umfassend
- eine Sammeleinrichtung (112, 212) zur Aufnahme und zur Vermischung der Insekten oder der Würmer mit Wasser zu einer Mischung;
- eine erste Fördereinrichtung (113, 213) zum Fördern der Mischung aus der Sammeleinrichtung (112, 212);
- eine erste Larvenseparierungseinrichtung (120, 220) zur Separierung der Insekten oder der Würmer vom Wasser aus der von der ersten Fördereinrichtung (113, 213) geförderten Mischung, wobei das separierte Wasser zur weiteren Verwendung in einer Insektenanlage, insbesondere in der Reinigungsanlage zur Verfügung steht, insbesondere zumindest teilweise in der Reinigungsanlage rezirkuliert wird.

2. Reinigungsanlage nach Anspruch 1, aufweisend
- zumindest einen Speichertank (130, 132, 230) zur Massenspeicherung der Mischung, wobei der zumindest eine Speichertank (130, 132, 230) bevorzugt nach der ersten Fördereinrichtung (113, 213) angeordnet ist, wobei die Reinigungsanlage vorzugsweise ferner aufweist:
- zumindest eine weitere Fördereinrichtung (121, 221; 231) zum Fördern der Mischung,
wobei die zumindest eine weitere Fördereinrichtung (121, 221; 231) vorzugsweise zwischen der ersten Fördereinrichtung (113, 213) und dem Speichertank (130, 132, 230) oder nach dem Speichertank (130, 132, 230) angeordnet ist.

3. Reinigungsanlage nach Anspruch 1 oder 2, aufweisend
- zumindest eine weitere Sammeleinrichtung zur Aufnahme und zur Vermischung der Insekten oder der Würmer mit Wasser zu einer Mischung, wobei die zumindest eine weitere Sammeleinrichtung vorzugsweise nach der ersten Larvenseparierungseinrichtung (120, 220) angeordnet ist.

4. Reinigungsanlage nach einem der vorangehenden Ansprüche, aufweisend
- zumindest eine weitere Larvenseparierungseinrichtung (240) zur weiteren Separierung der Insekten oder der Würmer und vom Wasser aus der geförderten Mischung, wobei vorzugsweise das separierte Wasser zur weiteren Verwendung in der Insektenanlage, insbesondere in der Reinigungsanlage zur Verfügung steht, insbesondere zumindest teilweise in der Reinigungsanlage rezirkuliert wird,
wobei die zumindest eine weitere Larvenseparierungseinrichtung (240) vorzugsweise nach der ersten Larvenseparierungseinrichtung (120, 220) angeordnet ist.

5. Reinigungsanlage nach einem der vorangehenden Ansprüche,
wobei zumindest eine weitere Wasserzuführung (122, 222) zur Nachwässerung der Mischung vorgesehen ist, die vorzugsweise nach der ersten Larvenseparierungseinrichtung (120, 220) in den Kreislauf und/oder in die weitere Sammeleinrichtung und/oder in den zumindest einen Speichertank mündet, und/oder
wobei vorzugsweise zumindest ein Wassertank (144, 244) zur Speicherung von Wasser aus und zur Abgabe von Wasser in den Kreislauf vorgesehen ist, und/oder
wobei vorzugsweise zumindest eine Filtereinrichtung (242, 142) vorgesehen ist zur Filterung des separierten Wassers aus der ersten Larvenseparierungseinrichtung (120, 220) und/oder der zumindest einen weiteren Larvenseparierungseinrichtung (240).

6. Reinigungsanlage nach einem der vorangehenden Ansprüche,
wobei zumindest eine Temperiereinrichtung (145, 246) zur Temperierung des Wassers vorgesehen ist, wobei die zumindest eine Temperiereinrichtung (145, 246) vorzugsweise zwischen dem zumindest einen Wassertank (144, 244) und der Sammeleinrichtung (112, 212) oder an dem Wassertank (144, 244) angeordnet ist, und/oder
wobei die Reinigungsanlage ferner eine Wiegeeinrichtung (111, 211) zur Bestimmung des Gewichts der Insekten oder Würmer aufweist, wobei die Wiegeeinrichtung (111, 112) vorzugsweise vor der Sammeleinrichtung (112, 212) oder in der Sammeleinrichtung (112, 212) integriert angeordnet ist.

7. Verfahren zur Reinigung von lebenden Insekten, insbesondere von lebenden Insektenlarven, oder von lebenden Würmern mit einer Reinigungsanlage nach einem der vorangehenden Ansprüche, umfassend die Schritte:
- Zuführen der lebenden Insekten oder lebenden Würmer in die Sammeleinrichtung (112, 212);
- erstes Vermischen der lebenden Insekten oder lebenden Würmer mit Wasser, bis eine bestimmte Mischung erreicht ist;
- Fördern der Mischung zu der ersten Larvenseparierungseinrichtung (120, 220) mittels der ersten Fördereinrichtung (113, 213);
- erstes Separieren der Mischung in der ersten Larvenseparierungseinrichtung (120, 220);
- Zumindest partielles Bereitstellen bzw. Rezirkulieren des separierten Wassers für die weitere Verwendung in der Insektenanlage, insbesondere in der Reinigungsanlage.

8. Verfahren nach Anspruch 7, weiter umfassend
- Fördern der in der ersten Larvenseparierungseinrichtung (120, 220) separierten Mischung zu dem zumindest einen Speichertank (130, 132, 230) mittels der zweiten Fördereinrichtung (121, 221);
- Speichern der Mischung in dem zumindest einen Speichertank (130, 132, 230).

9. Fördereinrichtung (113, 121, 213, 221, 231) zur Förderung von lebenden Insekten, insbesondere von lebenden Insektenlarven, oder von lebenden Würmern, in einer Reinigungsanlage nach einem Ansprüche 1 bis 6, wobei die Fördereinrichtung (113, 121, 213, 221, 231) als Kontaktlospumpe ausgebildet ist, wobei die Kontaktlospumpe insbesondere eine Peristaltikpumpe ist.

10. Larvenseparierungseinrichtung (120, 220, 240) zur Separierung von lebenden Insekten, insbesondere von lebenden Insektenlarven, oder von lebenden Würmern von Wasser, in einer Reinigungsanlage nach einem Ansprüche 1 bis 6, aufweisend
eine Siebtrommel mit einem Mantelabschnitt, der Maschen mit einer Maschenbreite von 1,0 mm bis 2,5 mm, vorzugsweise von 1,3 mm bis 2,0 mm, aufweist, und
mit einer Transportschraube (300) innerhalb der Siebtrommel zum Transportieren der Insekten oder der Würmer von einem Eintrittsende der Siebtrommel bis zu einem Austrittsende der Siebtrommel.

11. Larvenseparierungseinrichtung nach Anspruch 10,
wobei der Mantelabschnitt der Siebtrommel ein Keildraht-Sieb ist, und
wobei die Siebtrommel vorzugsweise einen Innendurchmesser von 500 mm bis 3000 mm, bevorzugt 1000 mm bis 1500 mm, besonders bevorzugt von 1100 mm bis 1250 mm, aufweist, und/oder
wobei die Transportschraube (300) eine Schraubenlänge aufweist, die sich über die gesamte Länge der Siebtrommel erstreckt, und/oder
wobei die Transportschraube (300) eine Schraubenhöhe von 5 mm bis 300 mm, vorzugsweise 50 mm bis 150 mm, besonders bevorzugt von 70 mm bis 100 mm, aufweist, und/oder
wobei die Transportschraube (300) eine Steigung von 200 mm bis 600 mm, bevorzugt 300 mm bis 400 mm, besonders bevorzugt von 330 mm bis 375 mm, aufweist.

12. Larvenseparierungseinrichtung nach einem der Ansprüche 10 oder 11,
wobei zumindest ein Spraybalken (400) zum Sprühen einer Flüssigkeit mittels mit vorzugsweise mehreren Düsen vorgesehen ist, wobei
der zumindest eine Spraybalken (400) 5 bis 40 Düsen, vorzugsweise 8 bis 28 Düsen, aufweist,
wobei vorzugsweise der zumindest eine Spraybalken (400) innerhalb der Siebtrommel angeordnet ist und/oder
der zumindest eine Spraybalken außerhalb der Siebtrommel angeordnet ist.

13. Larvenseparierungseinrichtung nach einem der Ansprüche 10 bis 12,
wobei zumindest ein Reinigungselement zur Reinigung des Mantelabschnitts der Siebtrommel vorgesehen ist,
wobei das zumindest eine Reinigungselement vorzugsweise zur Reinigung der Aussenseite der Siebtrommel ausgebildet ist und/oder
wobei das zumindest eine Reinigungselement als Bürstenelement ausgebildet ist,
wobei sich das zumindest eine Reinigungselement vorzugsweise über die gesamte Länge der Siebtrommel erstreckt.

14. Verfahren zur Separierung von lebenden Insekten, insbesondere von lebenden Insektenlarven, oder von lebenden Würmern von Wasser mittels einer Larvenseparierungseinrichtung (120, 220, 240) nach einem der Ansprüche 10 bis 13, umfassend die Schritte:
- Zuführen einer Mischung aus Insekten, insbesondere Insektenlarven, oder Würmern und Wasser, wobei die Mischung eine Temperatur von 5 °C bis 30 °C, vorzugsweise von 8 °C bis 15 °C oder 20 °C bis 26 °C aufweist, wobei
das Zuführen vorzugsweise kontinuierlich erfolgt,
- Drehen der Siebtrommel, wobei die Mischung entwässert wird und die Insekten oder die Würmer zur Austrittsöffnung transportiert werden.

15. Verfahren nach Anspruch 14,
wobei die Siebtrommel eine Drehzahl von 2 bis 60 Umdrehungen pro Minute, vorzugsweise 5 bis 20 Umdrehungen pro Minute, besonders bevorzugt eine Drehzahl von 10 bis 15 Umdrehungen pro Minute, aufweist, und/oder
wobei die lebenden Insekten oder die lebenden Würmer während dem Transport in der Siebtrommel mit Wasser aus dem zumindest einen Sprühbalken (400), vorzugsweise von zumindest einem innenseitig von der Siebtrommel angeordneten Sprühbalken (400), besprüht werden, wobei das Besprühen vorzugsweise kontinuierlich erfolgt und/oder mit einem Wasserduck von 2 bar bis 10 bar, vorzugsweise von 3 bar bis 7 bar erfolgt, und/oder
wobei zur Reinigung der Siebtrommel diese mit dem zumindest einen Sprühbalken (400), vorzugsweise mit einem außenseitig von der Siebtrommel angeordneten Sprühbalken (400), während der Rotation der Siebtrommel mit einer Flüssigkeit besprüht wird und/oder
wobei zur Reinigung der Siebtrommel diese von dem zumindest einen Reinigungselement während der Rotation der Siebtrommel kontaktiert wird.

## Claims

1. A cleaning system for cleaning live insects, in particular live insect larvae, or live worms comprising
- a collecting device (112, 212) for receiving and for mixing the insects or the worms with water to form a mixture;
- a first conveying device (113, 213) for conveying the mixture from the collecting device (112, 212);
- a first larvae separating device (120, 220) for separating the insects or the worms from the water of the mixture conveyed by the first conveying device (113, 213), wherein the separated water is available for further use in an insect system, in particular in the cleaning system, in particular is at least partially recirculated in the cleaning system.

2. The cleaning system according to claim 1, comprising
- at least one storage tank (130, 132, 230) for mass storage of the mixture, wherein the at least one storage tank (130, 132, 230) is preferably arranged downstream of the first conveying device (113, 213), wherein the cleaning system preferably further comprises:
- at least one further conveying device (121, 221; 231) for conveying the mixture,
wherein the at least one further conveying device (121, 221; 231) is preferably arranged between the first conveying device (113, 213) and the storage tank (130, 132, 230) or downstream of the storage tank (130, 132, 230).

3. The cleaning system according to claim 1 or 2, comprising
- at least one further collecting device for receiving and for mixing the insects or the worms with water to form a mixture, wherein the at least one further collecting device is preferably arranged downstream of the first larvae separating device (120, 220).

4. The cleaning system according to any one of the preceding claims, comprising
- at least one further larvae separating device (240) for further separating the insects or the worms and the water of the conveyed mixture, wherein preferably the separated water is available for further use in the insect system, in particular in the cleaning system, in particular is at least partially recirculated in the cleaning system,
wherein the at least one further larvae separating device (240) is preferably arranged downstream of the first larvae separating device (120, 220).

5. The cleaning system according to any one of the preceding claims,
wherein at least one further water supply (122, 222) is provided for post-watering of the mixture, which preferably opens into the circuit and/or into the further collecting device and/or into the at least one storage tank downstream of the first larvae separating device (120, 220), and/or
wherein preferably at least one water tank (144, 244) is provided for storing water from and releasing water into the circuit, and/or
wherein preferably at least one filter device (242, 142) is provided for filtering the separated water from the first larvae separating device (120, 220) and/or the at least one further larvae separating device (240).

6. The cleaning system according to any one of the preceding claims,
wherein at least one temperature control device (145, 246) is provided for controlling the temperature of the water, wherein the at least one temperature control device (145, 246) is preferably arranged between the at least one water tank (144, 244) and the collecting device (112, 212) or on the water tank (144, 244), and/or
wherein the cleaning system further comprises a weighing device (111, 211) for determining the weight of the insects or worms, wherein the weighing device (111, 211) is preferably arranged upstream of the collecting device (112, 212) or integrated in the collecting device (112, 212).

7. A method of cleaning live insects, in particular live insect larvae, or live worms by means of a cleaning system according to any one of the preceding claims, comprising the steps of:
- supplying the live insects or live worms into the collecting device (112, 212);
- first mixing of the live insects or live worms with water until a certain mixture is reached;
- conveying the mixture to the first larvae separating device (120, 220) by means of the first conveying device (113, 213);
- first separating of the mixture in the first larvae separating device (120, 220);
- at least partial provision or recirculation of the separated water for further use in the insect system, in particular in the cleaning system.

8. The method according to claim 7, further comprising
- conveying the mixture separated in the first larvae separating device (120, 220) to the at least one storage tank (130, 132, 230) by means of the second conveying device (121, 221);
- storing the mixture in the at least one storage tank (130, 132, 230).

9. A conveying device (113, 121, 213, 221, 231) for conveying live insects, in particular live insect larvae, or live worms in a cleaning system according to any one of claims 1 to 6, wherein the conveying device (113, 121, 213, 221, 231) is configured as a contactless pump, wherein the contactless pump is in particular a peristaltic pump.

10. A larvae separating device (120, 220, 240) for separating live insects, in particular live insect larvae, or live worms from water in a cleaning system according to any one of claims 1 to 6, comprising
a screening drum having a shell portion comprising meshes having a mesh width of from 1.0 mm to 2.5 mm, preferably from 1.3 mm to 2.0 mm, and
having a transport screw (300) within the screening drum for transporting the insects or the worms from an inlet end of the screening drum to an outlet end of the screening drum.

11. The larvae separating device according to claim 10,
wherein the shell portion of the screening drum is a wedge wire screen, and
wherein the screening drum preferably has an inner diameter of 500 mm to 3000 mm, preferably 1000 mm to 1500 mm, particularly preferably 1100 mm to 1250 mm, and/or
wherein the transport screw (300) has a screw length that extends along the entire length of the screening drum, and/or
wherein the transport screw (300) has a screw height of from 5 mm to 300 mm, preferably from 50 mm to 150 mm, particularly preferably from 70 mm to 100 mm, and/or
wherein the transport screw (300) has a pitch of from 200 mm to 600 mm, preferably from 300 mm to 400 mm, particularly preferably from 330 mm to 375 mm.

12. The larvae separating device according to any one of claims 10 or 11,
wherein at least one spray bar (400) is provided for spraying a liquid by means of preferably several nozzles, wherein
the at least one spray bar (400) has 5 to 40 nozzles, preferably 8 to 28 nozzles,
wherein preferably the at least one spray bar (400) is arranged inside the screening drum and/or
the at least one spray bar is arranged outside the screening drum.

13. The larvae separating device according to any one of claims 10 to 12,
wherein at least one cleaning element is provided for cleaning the shell portion of the screening drum,
wherein the at least one cleaning element is preferably configured for cleaning the outside of the screening drum and/or
wherein the at least one cleaning element is configured as a brush element,
wherein the at least one cleaning element preferably extends along the entire length of the screening drum.

14. A method for separating live insects, in particular live insect larvae, or live worms from water by means of a larvae separating device (120, 220, 240) according to any one of claims 10 to 13, comprising the steps of:
- supplying a mixture of insects, in particular insect larvae, or worms and water, wherein the mixture has a temperature of from 5°C to 30°C, preferably from 8°C to 15°C or 20°C to 26°C, wherein
the supplying preferably takes place continuously,
- rotating the screening drum, wherein the mixture is dehydrated and the insects or the worms are transported to the outlet opening.

15. The method according to claim 14,
wherein the screening drum has a rotational speed of 2 to 60 revolutions per minute, preferably 5 to 20 revolutions per minute, particularly preferably a rotational speed of 10 to 15 revolutions per minute, and/or
wherein the live insects or the live worms are sprayed with water from the at least one spray bar (400), preferably from at least one spray bar (400) arranged on the inside of the screening drum, during the transport in the screening drum, wherein the spraying preferably takes place continuously and/or takes place with a water pressure of 2 bar to 10 bar, preferably of 3 bar to 7 bar, and/or
wherein, for cleaning the screening drum, the latter is sprayed with a liquid during the rotation of the screening drum by means of the at least one spray bar (400), preferably by means of a spray bar (400) arranged on the outside of the screening drum, and/or
wherein, for cleaning the screening drum, the latter is contacted by the at least one cleaning element during the rotation of the screening drum.

## Revendications

1. Installation de nettoyage pour le nettoyage d'insectes vivants, en particulier de larves d'insectes vivantes ou de vers vivants, comprenant
- un dispositif de collecte (112, 212) pour la réception et le brassage des insectes ou des vers avec de l'eau pour former un mélange ;
- un premier dispositif de transport (113, 213) pour le transport du mélange hors du dispositif de collecte (112, 212) ;
- un premier dispositif de séparation des larves (120, 220) pour la séparation des insectes ou des vers de l'eau du mélange transporté par le premier dispositif de transport (113, 213), l'eau séparée étant disponible pour une autre utilisation dans une installation pour insectes, en particulier dans l'installation de nettoyage, et étant en particulier remise au moins partiellement en circulation dans l'installation de nettoyage.

2. Installation de nettoyage selon la revendication 1, comprenant
- au moins un réservoir de stockage (130, 132, 230) pour le stockage du mélange, ledit au moins un réservoir de stockage (130, 132, 230) étant préférentiellement disposé en aval du premier dispositif de transport (113, 213), ladite installation de nettoyage comprenant préférentiellement en outre :
- au moins un autre dispositif de transport (121, 221 ; 231) pour le transport du mélange, ledit au moins un autre dispositif de transport (121, 221 ; 231) étant préférentiellement disposé entre le premier dispositif de transport (113, 213) et le réservoir de stockage (130, 132, 230) ou en aval du réservoir de stockage (130, 132, 230).

3. Installation de nettoyage selon la revendication 1 ou la revendication 2, comprenant
- au moins un autre dispositif de collecte pour la collecte et le brassage des insectes ou des vers avec de l'eau pour former un mélange, ledit au moins un autre dispositif de collecte étant préférentiellement disposé en aval du premier dispositif de séparation des larves (120, 220).

4. Installation de nettoyage selon l'une des revendications précédentes, comprenant
- au moins un autre dispositif de séparation des larves (240) pour une autre séparation des insectes ou des vers et de l'eau du mélange transporté, l'eau séparée étant préférentiellement disponible pour une autre utilisation dans l'installation pour insectes, en particulier dans l'installation de nettoyage, et étant en particulier remise au moins partiellement en circulation dans l'installation de nettoyage,
où ledit au moins un autre dispositif de séparation des larves (240) est préférentiellement disposé en aval du premier dispositif de séparation des larves (120, 220).

5. Installation de nettoyage selon l'une des revendications précédentes,
où au moins une autre alimentation en eau (122, 222) est prévue pour le trempage consécutif du mélange, laquelle débouche préférentiellement en aval du premier dispositif de séparation des larves (120, 220) dans le circuit et/ou dans l'autre dispositif de collecte et/ou dans ledit au moins un réservoir de stockage, et/ou
où au moins un réservoir d'eau (144, 244) est préférentiellement prévu pour le stockage de l'eau et pour le refoulement d'eau dans le circuit, et/ou
où au moins un dispositif de filtre (242, 142) est préférentiellement prévu pour le filtrage de l'eau séparée du premier dispositif de séparation des larves (120, 220) et/ou dudit au moins un autre dispositif de séparation des larves (240).

6. Installation de nettoyage selon l'une des revendications précédentes,
où au moins un dispositif de régulation de température (145,246) est prévu pour la régulation de température de l'eau, où ledit au moins un dispositif de régulation de température (145, 246) est préférentiellement disposé entre ledit au moins un réservoir d'eau (144, 244) et le dispositif de collecte (112, 212) ou contre le réservoir d'eau (144, 244), et/ou
où l'installation de nettoyage comprend en outre un dispositif de pesage (111, 211) pour la détermination du poids des insectes ou des vers, ledit dispositif de pesage (111, 211) étant préférentiellement disposé en amont du dispositif de collecte (112, 212) ou intégré au dispositif de collecte (112, 212).

7. Procédé de nettoyage d'insectes vivants, en particulier de larves d'insectes vivantes, ou de vers vivants avec une installation de nettoyage selon l'une des revendications précédentes, comprenant les étapes suivantes :
- amenée des insectes vivants ou des vers vivants dans le dispositif de collecte (112, 212) ;
- premier brassage des insectes vivants ou des vers vivants avec l'eau, jusqu'à obtention d'un mélange défini ;
- transport du mélange vers le premier dispositif de séparation des larves (120, 220) au moyen du premier dispositif de transport (113, 213) ;
- première séparation du mélange dans le premier dispositif de séparation des larves (120, 220) ;
- fourniture ou recirculation au moins partielle de l'eau séparée pour une autre utilisation dans l'installation pour insectes, en particulier dans l'installation de nettoyage.

8. Procédé selon la revendication 7, comprenant en outre
- transport du mélange séparé dans le premier dispositif de séparation des larves (120,220) vers ledit au moins un réservoir de stockage (130, 132, 230) au moyen du deuxième dispositif de transport (121, 221) ;
- stockage du mélange dans ledit au moins un réservoir de stockage (130, 132, 230).

9. Dispositif de transport (113, 121, 213, 221, 231) pour le transport d'insectes vivants, en particulier de larves d'insectes vivantes ou de vers vivants, dans une installation de nettoyage selon l'une des revendications 1 à 6, ledit dispositif de transport (113, 121, 213, 221, 231) étant réalisé comme pompe sans contact, ladite pompe sans contact étant en particulier une pompe péristaltique.

10. Dispositif de séparation des larves (120, 220, 240) pour la séparation d'insectes vivants, en particulier de larves d'insectes vivantes ou de vers vivants de l'eau, dans une installation de nettoyage selon l'une des revendications 1 à 6, comprenant
un tambour de tamisage avec une partie de panneau, présentant un maillage avec une largeur de maille comprise entre 1,0 mm et 2,5 mm, préférentiellement entre 1,3 mm et 2,0 mm, et
avec une vis de transport (300) à l'intérieur du tambour de tamisage pour le transport des insectes ou des vers d'une extrémité d'entrée du tambour de tamisage à une extrémité de sortie du tambour de tamisage.

11. Dispositif de séparation des larves selon la revendication 10,
où la partie de panneau du tambour de tamisage est un tamis en toile métallique, et
où le tambour de tamisage présente préférentiellement un diamètre intérieur compris entre 500 mm et 3000 mm, avantageusement entre 1000 mm et 1500 mm, préférentiellement entre 1100 mm et 1250 mm, et/ou
où la vis de transport (300) a une longueur de vis s'étendant sur toute la longueur du tambour de tamisage, et/ou
où la vis de transport (300) a une hauteur de vis comprise entre 5 mm et 300 mm, avantageusement entre 50 mm et 150 mm, préférentiellement entre 70 mm et 100 mm, et/ou
où la vis de transport (300) a un pas compris entre 200 mm et 600 mm, avantageusement entre 300 mm et 400 mm, préférentiellement entre 330 mm et 375 mm.

12. Dispositif de séparation des larves selon la revendication 10 ou la revendication 11,
où au moins une barre de pulvérisation (400) est prévu pour la pulvérisation d'un liquide préférentiellement au moyen de plusieurs buses, où
ladite au moins une barre de pulvérisation (400) comprend entre 5 et 40 buses, préférentiellement entre 8 et 28 buses,
où ladite au moins une barre de pulvérisation (400) est préférentiellement disposée à l'intérieur du tambour de tamisage et/ou
ladite au moins une barre de pulvérisation est disposée à l'extérieur du tambour de tamisage.

13. Dispositif de séparation des larves selon l'une des revendications 10 à 12,
où au moins un élément de nettoyage est prévu pour le nettoyage de la partie de panneau du tambour de tamisage,
où ledit au moins un élément de nettoyage est préférentiellement conçu pour le nettoyage de la surface extérieure du tambour de tamisage et/ou
où ledit au moins un élément de nettoyage est réalisé comme élément de brosse, ledit au moins un élément de nettoyage s'étendant préférentiellement sur toute la longueur du tambour de tamisage.

14. Procédé de séparation d'insectes vivants, en particulier de larves d'insectes vivantes ou de vers vivants, de l'eau au moyen d'un dispositif de séparation des larves (120, 220, 240) selon l'une des revendications 10 à 13, comprenant les étapes suivantes :
- amenée d'un mélange d'insectes, en particulier de larves d'insectes, ou de vers et d'eau, ledit mélange ayant une température comprise entre 5 °C et 30 °C, préférentiellement entre 8 °C et 15 °C ou entre 20 °C et 26 °C, où
l'amenée est préférentiellement continue,
- rotation du tambour de tamisage, le mélange étant déshydraté et les insectes ou les vers transportés vers l'orifice de sortie.

15. Procédé selon la revendication 14,
où le tambour de tamisage a une vitesse de rotation comprise entre 2 et 60 tours/minute, avantageusement entre 5 et 20 tours/minute, préférentiellement entre 10 et 15 tours/minute, et/ou
où les insectes vivants ou les vers vivants sont aspergés d'eau par ladite au moins une barre d'aspersion (400) pendant le transport dans le tambour de tamisage, préférentiellement d'eau par au moins une barre d'aspersion (400) disposée à l'intérieur du tambour de tamisage, l'aspersion étant préférentiellement continue et/ou étant effectuée avec une pression d'eau comprise entre 2 bar et 10 bar, préférentiellement entre 3 bar et 7 bar, et/ou
où, pour le nettoyage du tambour de tamisage, celui-ci est aspergé d'un liquide par ladite au moins une barre d'aspersion (400), préférentiellement par une barre d'aspersion (400) disposée extérieurement au tambour de tamisage, pendant la rotation du tambour de tamisage, et/ou
où, pour le nettoyage du tambour de tamisage, celui-ci est mis en contact avec ledit au moins un élément de nettoyage pendant la rotation du tambour de tamisage.
